# EUROPÄISCHE PATENTSCHRIFT

(11) **EP 3 365 421 B1**
(45) Veröffentlichungstag und Bekanntmachung des Hinweises auf die Patenterteilung: **21.04.2021**
(21) Anmeldenummer: 16781432.6
(22) Anmeldetag: 13.10.2016
(51) Int. Cl.: C11D 3/50, A23L 27/00, A61L 9/01, A61Q 13/00, A61K 8/02, A61K 9/20, C11D 3/37, A61K 8/81

(54) **FESTE LÖSUNGEN VON GERUCHS- UND GESCHMACKSSTOFFEN MIT VINYLLACTAMPOLYMEREN**
SOLID SOLUTIONS OF ODORIFEROUS SUBSTANCES AND FLAVORING AGENTS WITH VINYL LACTAM POLYMERS
SOLUTIONS SOLIDES DE SUBSTANCES ODORANTES ET AROMATISANTES CONTENANT DES POLYMÈRES DE LACTAMES VINYLIQUES

(30) Priorität: 23.10.2015 EP 15191156
(43) Veröffentlichungstag der Anmeldung: 29.08.2018
(73) Patentinhaber: BASF SE, 67056 Ludwigshafen am Rhein (DE)
(72) Erfinder: KOLTER, Karl, 67117 Limburgerhof (DE); PELZER, Ralf, 37699 Fuerstenberg (DE); KARL, Matthias, 68163 Mannheim (DE)
(74) Vertreter: BASF IP Association
(86) Internationale Anmeldenummer: PCT/EP2016/074566
(87) Internationale Veröffentlichungsnummer: WO 2017/067841

(56) Entgegenhaltungen:
- EP-A2- 0 998 919
- EP-A2- 2 463 327
- WO-A1-2011/033085
- US-A- 4 448 789
- US-B1- 6 423 256

## Beschreibung

Die vorliegende Erfindung betrifft wasserlösliche Formulierungen von Geruchs- und Geschmacksstoffen, in denen die Geruchs- und Geschmacksstoffe homogen in einer Polymermatrix auf Basis von Polyvinyllactamen eingebettet vorliegen. Weiterhin betrifft die Erfindung die Verwendung solcher Formulierungen.

Die Formulierung von Geruchs- und Geschmacksstoffen ist aufgrund der physikochemischen Eigenschaften dieser Stoffe nicht einfach. Zum einen sind sie vielfach stark flüchtig, was eine Verarbeitung bei höherer Temperatur erschwert und zum anderen sind sie oft in Wasser schwerlöslich, wodurch sie nur in unzureichender Menge in wässrige Systeme eingearbeitet werden können. Aufgrund ihrer Diffusivität diffundieren sie in kompartimentierten Formulierungen in Bereiche, in denen sie nicht oder nicht in der gewünschten Konzentration vorhanden sein sollten. Dies stellt auch ein Problem bei sogenannten mikroverkapselten Systemen dar. Die gesteuerte Freisetzung von Geruchs- und Geschmacksstoffen ist generell ein schwieriges und nicht zufriedenstellend gelöstes Problem.

Häufig werden fett- und wachsartige Substanzen für die Formulierung von Geruchs- und Geschmacksstoffen verwendet wie in US 2005/0227905 beschrieben.

Allerdings haben diese den Nachteil, dass sie einen niedrigen Schmelzpunkt aufweisen und somit temperatur- und druckempfindlich sind. Zudem sind diese Stoffe nicht in Wasser löslich, so dass bei der Auflösung in wässrigen Medien wenig Geruchs- und Geschmacksstoffe in die wässrige Phase übergehen und auch unschöne feste Rückstände zurückbleiben.

Ähnlich verhält es sich mit den in der US 2009/0301504 A1 beschriebenen Formulierungen, die ebenfalls Wachse, Fette, Sterole oder Polyethylenglykol in Kombination mit weiteren Stoffen wie Surfactants beschreibl.

WO 2011/033085 A1 offenbart wasserlösliche Extrudate, die aus solubilisierend wirkenden Copolymeren (z.B. Terpolymere aus N-Vinyllactam, Vinylacetat und Polyether) und weiteren Polymeren, die geeignet sind die Freisetzung der biologisch aktiven Substanz zu beeinflussen bestehen.

Ein weiteres Problem besteht in der Stabilität der Formulierungen gegen Umwelteinflüsse. Häufig kommt es zu unerwünschten Verfärbungen durch Zersetzung der Geruchs- und Geschmacksstoffe. Ebenso unerwünscht ist eine Phasenseparation der Formulierungen, die zu einem Ausbluten der Geruchs- und Geschmacksstoffe führen kann.

Hinzu kommt, dass sich die Geruchs- und Geschmacksstoffe aufgrund ihrer physikalischen Eigenschaften oft nicht gut verarbeiten lassen. Häufig entstehen stark klumpende Massen.

Aufgabe der vorliegenden Erfindung war es, wasserlösliche Formulierungen von Geruchs- und Geschmacksstoffen zu finden, die eine zeitlich kontrollierte Freisetzung der Geruchsstoffe erlauben. Weiterhin sollten die Formulierungen auch bei der Verarbeitung gut handhabbar sein. Die Formulierungen sollten auch stabil gegen Umwelteinflüsse, um unerwünschte Verfärbungen oder Veränderungen des Geruchs- oder Geschmacksprofils zu vermeiden, um möglichst glasklare Formulierungen mit einem geringen Farbwert zu erhalten. Weiterhin sollten die Geruchs-und Geschmacksstoffe auch gegen ein Ausbluten aus der Formulierung beziehungsweise Diffusion in unerwünschte Bereiche geschützt sein.

Demgemäß wurden optisch klare, feste wasserlösliche Formulierungen von Geruchs- und Geschmacksstoffen, enthaltend mindestens einen synthetischen Geschmacksstoff, gefunden, in denen die Geruchs- und Geschmacksstoffe homogen in einer Polymermatrix auf Basis eines Propfcopolymers aus (i) 50 bis 60 Gew.-% N-Vinyllactam, bevorzugt N-Vinylcaprolactam, (ii) 25 bis 35 Gew.-% Vinylacetat, und (iii) 10 bis 20 Gew.-% eines Polyethers, bevorzugt Polyethylenglykol, mitder Maßgabe, dass die Summe der Komponenten i), ii), und iii) gleich 100 Gew.-% beträgt, eingebettet vorliegen.

Erfindungsgemäß bedeutet "wasserlöslich", dass unter Standardbedingungen (20 °C, 0.101325 MPa) 10 g Substanz/ Liter Wasser gelöst werden können.

Die erfindungsgemäßen Formulierungen sind optisch klar, was bedeutet, dass unter einem Lichtmikroskop mit einer 40fachen Vergrößerung keine Phasentrennung zu beobachten ist.

Weiterhin sind die erfindungsgemäßen Formulierungen farblos oder nur leicht gefärbt. Das bedeutet, dass der Farbwert einer 1%igen Zubereitung der Formulierung in Wasser gemäß der Europäischen Pharmakopöe nicht größer als B5, BY5, Y5, GY5, R5 beträgt. Vorzugsweise ist die Farbe nicht intensiver als B6, BY6, Y6, GY6, R6.

Vorzugsweise liegen die Formulierungen in Form fester Lösungen der Geruchs- und Geschmacksstoffe in der Polymermatrix auf Basis von Polyvinyllactamen vor. Der Begriff "feste Lösungen" ist dem Fachmann bekannt und bezeichnet eine molekulardisperse Einbettung der Geruchs- und Geschmacksstoffe in der Polymermatrix.

Es können Geruchs und Geschmacksstoffe unterschiedlicher Art und Anzahl eingearbeitet werden, wobei erfindungsgemäß mindestens ein synthetischer Geruchsstoff in den Formulierungen enthalten ist. Dabei können einzelne synthetische Geruchsstoffe wie z.B. Geraniol, Linalool, Linalylacetat, Pyranol, Geranylacetat, Anisaldehyd, Citral, Citronellal, Lysmeral, Citronellol, Rosenoxid, Tetrahydrolinalool, Hydroxycitronellal, Betaionon, Menthol, Zimtaldehyd, Anethol, Vanillin, Eugenol, Carvon, Piperonal eingesetzt werden. Weiterhin können neben einem synthetischen Geruchs- und Geschmacksstoff etherische Öle wie beispielsweise Zimtöl, bis zu komplexen Parfüm- oder Geschmackskompositionen zugesetzt werden. Besonders bevorzugt sind Linalool, Geraniol und Menthol.

Als Polyvinyllactame werden Pfropfcopolymere, die erhältlich sind aus
i) 50 bis 60 Gew.-% N-Vinyllactam, bevorzugt N-Vinylcaprolactam
ii) 25 bis 35 Gew.-% Vinylacetat, und
iii) 10 bis 20 Gew.-% eines Polyethers, bevorzugt Polyethylenglykol,
mit der Maßgabe, dass die Summe der Komponenten i), ii), und iii) gleich 100 Gew.-% beträgt, verwendet

Bevorzugt ist ein Pfropfcopolymer aus 13 Gew.-% Polyethylenglykol MG 6000, 57 Gew.-% N-Vinylcaprolactam und 30 Gew.-% Vinylacetat mit einem mittleren Molgewicht von 44.000 Dalton.

Allgemeine Verfahren zur Herstellung der erfindungsgemäßen Pfropf-Copolymerisate sind an sich bekannt. Die Herstellung erfolgt durch freie radikalisch initiierte Polymerisation, bevorzugt in Lösung, in nichtwässrigen, organischen Lösungsmitteln oder in gemischt nichtwässrigen/wässrigen Lösungsmitteln. Geeignete Herstellverfahren sind beispielsweise in der WO 2007/051743 und der WO 2009/013202 beschrieben.

Die Menge an Geruchs- und Geschmacksstoffen in der Polymermatrix richtet sich zum einen nach der Art der Geruchs- und Geschmacksstoffe, deren Intensität individuell sehr verschieden sein kann, und zum Anderen nach der Art der Anwendung der Formulierungen.

Als weitere Additive können verwendet werden: Farbstoffe und Pigmente, Weichmacher, Stabilisatoren, Konservierungsmittel, Emulgatoren, Solubilisatoren, oberflächenaktive Stoffe, Benetzungsmittel, Stoffe zur Viskositätseinstellung, Antioxidantien.

Die Herstellung der erfindungsgemäßen Zubereitungen erfolgt vorzugsweise über Schmelzextrusion, das heißt der Geruchs- und Geschmacksstoff wird zusammen mit dem Polymer über die Glasübergangstemperatur erhitzt, vermischt, geknetet und durch eine Düse gepresst. Aus den extrudierten Strängen, Bändern oder Folien können anschließend Granulate, Pulver, Folienstücke, das heißt vielfältige Formen hergestellt werden. Überraschenderweise entweichen die Geruchs- und Geschmacksstoffe während des Prozesses nicht und werden auch nicht zersetzt, obwohl dieser bei relativ hohen Temperaturen gefahren wird. Der übliche Temperaturbereich liegt zwischen 70 und 250°, vorzugsweise zwischen 80 und 200 und besonders bevorzugt zwischen 90 und 170°C.

Neben Extrudern können auch Kneter oder Spritzgießmaschinen eingesetzt werden. Als alternatives Herstellungsverfahren ist die Sprühtrocknung entweder aus wässriger oder organischer Lösung geeignet. Filmziehverfahren können ebenfalls eingesetzt werden.

Der Beladungsgrad der erfindungsgemäßen Zubereitungen an Geruchs- oder Geschmacksstoff liegt zwischen 1 und 50%, vorzugsweise zwischen 5 und 40% (m/m).

Überraschenderweise wurde gefunden, dass Polyvinyllactame in der Lage sind Geruchs- und Geschmacksstoffe aufzunehmen und zu binden, wobei in der Regel klare, glasartige Formulierungen entstehen, die vielfältig eingesetzt werden können und auch weiterverarbeitet werden können. Diese glasartigen Zubereitungen geben den Geruchs- und Geschmacksstoff verzögert ab. Ein besonderes Charakteristikum dieser glasartigen Zubereitungen ist, dass nahezu keine Porosität vorhanden ist und somit die scheinbare Dichte nahe der wahren Dichte liegt. Der Geruchs- und Geschmacksstoffen wird somit vollständig eingeschlossen und liegt extrem geschützt vor, insbesondere gegen Sauerstoff und Feuchtigkeit.

Wird die Zubereitung in ein wässriges Medium gebracht, wird der Geruchs- und Geschmacksstoff in die wässrige Phase überführt und in dieser auch über seiner Sättigungslöslichkeit in Wasser in Lösung gehalten. Durch eine Mizellbildung mit einem amphiphilen Polymer kann zudem ein starker Retardeffekt erzielt werden, das heißt der Geruchsstoff wird aus dem wässrigen Medium nur verzögert abgegeben oder in anderen Worten ausgedrückt, die Lösung riecht sehr lange. Die Geruchs- und Geschmacksstoffe weisen eine hohe Affinität zu den Polyvinyllactamen auf und können als feste Lösung inkorporiert werden.

Die resultierenden Zubereitungen sind in fester Form sehr stabil und die Polymere werden auch in wässriger Form nicht hydrolysiert.

Die Verwendung der erfindungsgemäßen Zubereitungen ist sehr vielfältig. So können sie als Wasch- und Geschirrspülmittelzusätze, als Kosmetikzusätze, in Pharmazeutika oder Nahrungsergänzungsmitteln, in Lebensmitteln, zur Abgabe von Duftstoffen in Räumen und in Autos, in Raumbefeuchtern, in Duftlösungen verwendet werden.

Die Freisetzung der Geruchs- und Geschmacksstoffe kann gemäß einer bevorzugten Ausführungsform der Erfindung durch Bestimmung des Gewichtsverlusts der Formulierung über die Zeit, insbesondere unter Temperaturbelastung, erfolgen.

Die Freisetzung von Geruchs- und Geschmacksstoffen, aus den erfindungsgemäßen Formulierungen kann auch durch die gaschromatographische Untersuchung des Dampfraums über Proben der entsprechenden Formulierungen verfolgt werden. Gemäß einer Ausführungsform kann die Bestimmung wie im Folgenden beschrieben erfolgen. In einer mit einem kontrollierten Gasstrom, z.B. Luft oder Stickstoff oder weitere Inerte Gase, mit kontrollierter Temperatur und Luftfeuchtigkeit, durchströmten temperierten Messkammer, in die Proben der Formulierung in geeigneter Weise eingebracht wurden, kann in bestimmten zeitlichen Abständen ein definiertes Volumen des Abgasstroms auf ein geeignetes Adsorbens geleitet werden. Der Gehalt an Geruchs-oder Geschmacksstoff kann anschliessend nach Thermodesorption gaschromatographisch bestimmt werden.

Gemäß einer anderen Ausführungsform werden Proben der erfindungsgemäßen Formulierung in geeigneter Weise in offene Rollrandgefäße eingebracht. Die Rollrandgefäße werden nach bestimmten Zeitabständen verschlossen und der verbliebene Geruchs- und Geschmacksstoffgehalt kann im Dampfraum über der Probemittels geeigneter Methoden wie Dampfraumchromatographie oder Festfasermikroextraktion mit anschliessender Gaschromatographie bestimmt werden."

### Beispiele

Als Polymer wurde ein kommerziell unter dem Namen Soluplus®, Firma BASF, erhältliches Pfropfcopolymer aus 13 Gew.-% Polyethylenglykol MG 6000, 57 Gew.-% N-Vinylcaprolactam und 30 Gew.-% Vinylacetat mit einem mittleren Molgewicht von 44.000 Dalton eingesetzt.

Die Schmelzextrusion erfolgte mittels eines Doppelschneckenextruders der Firma Thermofischer Pharma 11 Twin-screw Extruder, L/D-Verhältnis 40,Schneckendurchmesser 11 mm

Die Bestimmung der Klarheit erfolgte mittels eines Lichtmikroskops mit einer 40fachen Vergrößerung

Die Farbe wurde gemäß der Europäischen Pharmakopöe 8. Ausgabe bestimmt.

### Beispiel 1

Die Extrusion der Formulierung aus Polymer (Feststoff) mit 13 % (w/w) Linalool (Flüssigkeit) erfolgte unter den folgenden Bedingungen:
- Dosiereinheit Polymer: 300 g/h
- Flüssigdosierung Linalool: 45 g/h
- Zonentemperatur 5-7: 120°C
- Zonentemperatur 8: 130°C
- Schneckendrehzahl 200 UpM
- Düsendurchmesser 2 mm
- Düsendruck: 5 bar
- Drehmoment: 6,9 Nm

Aussehen: Klares, farbloses Extrudat, löslich in Wasser; beim Lösen in Wasser wird ein Geruch nach Linalool Geruch wahrnehmbar. Farbe nicht intensiver als B6, BY6, Y6, GY6, R6

### Beispiel 2

Die Extrusion der Formulierung aus Polymer (Feststoff) mit 22 % (w/w) Linalool (Flüssigkeit) erfolgte unter den folgenden Bedingungen:
- Dosiereinheit Polymer: 300 g/h
- Flüssigdosierung Linalool: 84 g/h
- Zonentemperatur 5-7: 120°C
- Zonentemperatur 8: 130°C
- Schneckendrehzahl 200 UpM
- Düsendurchmesser 2 mm
- Düsendruck: 2 bar
- Drehmoment: 6,9 Nm

Aussehen: Klares, farbloses Extrudat, löslich in Wasser; beim Lösen wird ein Geruch nach Linalool wahrnehmbar. Farbe nicht intensiver als B6, BY6, Y6, GY6, R6

### Beispiel 3

Die Extrusion der Formulierung aus Polymer (Feststoff) mit 7 % (w/w) Geraniol Extra (Flüssigkeit) erfolgte unter den folgenden Bedingungen:
- Dosiereinheit Polymer: 500 g/h
- Flüssigdosierung Geraniol Extra: 39 g/h
- Zonentemperatur 5-8: 130°C
- Schneckendrehzahl 200 UpM
- Düsendurchmesser 2 mm
- Düsendruck: 16 bar
- Drehmoment: 7,0 Nm

Aussehen: Klares, farbloses Extrudat, löslich in Wasser; beim Lösen in Wasser wird ein Geruch nach Geraniol Extra wahrnehmbar. Farbe nicht intensiver als B6, BY6, Y6, GY6, R6

### Beispiel 4

Die Extrusion der Formulierung aus Polymer (Feststoff) mit 13 % (w/w) Geraniol Extra (Flüssigkeit) erfolgte unter den folgenden Bedingungen:
- Dosiereinheit Polymer: 300 g/h
- Flüssigdosierung Geraniol Extra: 45 g/h
- Zonentemperatur 5-7: 120°C
- Zonentemperatur 8: 130°C
- Schneckendrehzahl 200 UpM
- Düsendurchmesser 2 mm
- Düsendruck: 11 bar
- Drehmoment: 7,3 Nm

Aussehen: Klares, farbloses Extrudat, löslich in Wasser; beim Lösen in Wasser wird ein Geruch nach Geraniol wahrnehmbar. Farbe nicht intensiver als B6, BY6, Y6, GY6, R6

### Beispiel 5

Die Extrusion der Formulierung aus Polymer (Feststoff) mit 20 % (w/w) Geraniol Extra (Flüssigkeit) erfolgte unter den folgenden Bedingungen:
- Dosiereinheit Polymer: 300 g/h
- Flüssigdosierung Geraniol Extra: 75 g/h
- Zonentemperatur 5-7: 120°C
- Zonentemperatur 8: 130°C
- Schneckendrehzahl 200 UpM
- Düsendurchmesser 2 mm
- Düsendruck: 8 bar
- Drehmoment: 7,0 Nm

Aussehen: Klares Extrudat, löslich in Wasser; beim Lösen in Wasser wird ein Geruch nach Geraniol wahrnehmbar. Farbe nicht intensiver als B6, BY6, Y6, GY6, R6

### Beispiel 6

Die Extrusion der Formulierung aus Polymer (Feststoff) mit 7 % (w/w) L-Menthol (Aufschmelzen des festen L-Menthol, mit anschließender flüssiger Dosierung in den Extrusionsprozess) erfolgte unter den folgenden Bedingungen:
- Dosiereinheit Polymer: 500 g/h
- Flüssigdosierung L-Menthol: 38 g/h
- Zonentemperatur 5-8: 140°C
- Schneckendrehzahl 200 UpM
- Düsendurchmesser 2 mm
- Düsendruck: 24 bar
- Drehmoment: 6,3 Nm

Aussehen: Klares Extrudat, löslich in Wasser; beim Lösen in Wasser wird ein Geruch nach L-Menthol wahrnehmbar. Farbe nicht intensiver als B6, BY6, Y6, GY6, R6

### Beispiel 7 (Vergleichsbeispiel)

Physikalische Mischungen aus Polymer und jeweils 5 Gew.-%, bezogen auf Polymergewicht, Geruchsstoff (L-Menthol (fest), Linalool (flüssig) und Geraniol Extra (flüssig)), wurden in einemTurbula-Mischer T 2C der Firma Willy A. Bachofen AG (Basel) bei 22 rpm über 10 min hergestellt. Hierbei wurde das Polymer vorgelegt und der Geruchsstoff portionsweise zugegeben.

Beim Mischen der Komponenten entstanden sehr stark klebende und verklumpte Massen. Eine homogene Formulierung konnte auf diese Weise nicht hergestellt werden

### Test 1

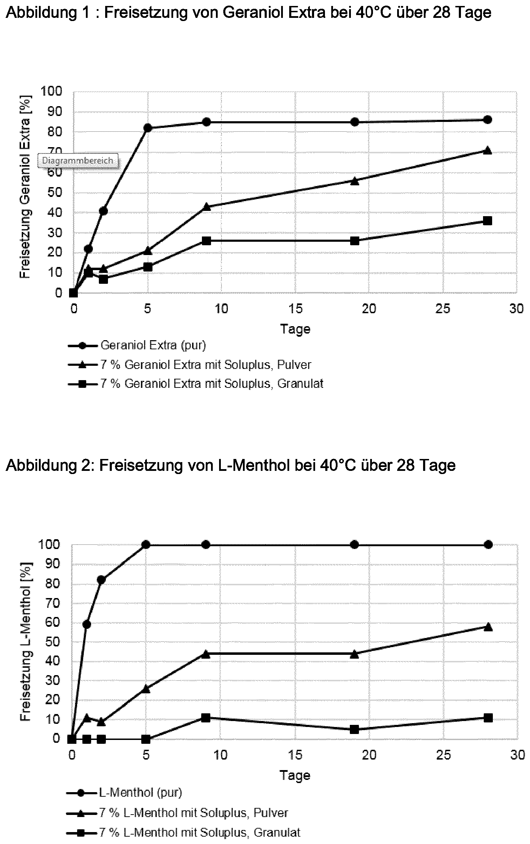

Bestimmung der Flüchtigkeit der Geruchs- und Geschmacksstoffe aus den Formulierungen bei 40°C:
Extrudiertes Polymer, Formulierungen und eine äquivalente Menge des Geruchsstoffs wurden in separate Trockenschalen eingewogen, bei konstanter Temperatur von 40°C eingelagert und an definierten Zeitpunkten den Masseverlust mit einer Analysenwaage bestimmt. Siehe auch Abbildungen 1 und 2.

| Formulierung | Anfang | 3 Tage | 7 Tag | 17 Tag | 26 Tag |
|---|---|---|---|---|---|
| Extrudiertes Soluplus Pulver (160°C, 200 rpm, 1 kg) | 100,0 | 100,7 | 100,2 | 100,6 | 100,4 |
| Extrudiertes Soluplus Granulat (160°C, 200 rpm, 1 kg) | 100,0 | 100,6 | 100,0 | 100,4 | 100,2 |

Umrechnung auf freigesetzten Geruch- und Geschmacksstoff nach Tagen:

| Formulierung | Anfang | 1 Tag | 2 Tage | 5 Tage | 9 Tage | 19 Tage | 28 Tage |
|---|---|---|---|---|---|---|---|
| Geraniol Extra (pur) | 0 mg | 33 mg | 62 mg | 123 mg | 127 mg | 128 mg | 129 mg |
| 7 % Geraniol Extra mit Soluplus, Pulver | 0 mg | 15 mg | 15 mg | 27 mg | 55 mg | 72 mg | 91 mg |
| 7 % Geraniol Extra mit Soluplus, Granulat | 0 mg | 12 mg | 8 mg | 16 mg | 32 mg | 32 mg | 44mg |
| L-Menthol (pur) | 0 mg | 83 mg | 115 mg | 141 mg | 141 mg | 141 mg | 141 mg |
| 7 % L-Menthol mit Soluplus Pulver | 0 mg | 12 mg | 10 mg | 29 mg | 50 mg | 50 mg | 65 mg |
| 7 % L-Menthol mit Soluplus Granulat | 0 mg | 0 mg | 0 mg | 0 mg | 14 mg | 7 mg | 14 mg |

| Formulierung | Anfang | 1 Tag | 2 Tage | 5 Tage | 9 Tage | 19 Tage | 28 Tage |
|---|---|---|---|---|---|---|---|
| Geraniol Extra (pur) | 0% | 22 % | 41 % | 82 % | 85 % | 85 % | 86 % |
| 7 % Geraniol Extra mit Soluplus, Pulver | 0 % | 12 % | 12 % | 21 % | 43 % | 56 % | 71 % |
| 7 % Geraniol Extra mit Soluplus, Granulat | 0 % | 10 % | 7 % | 13 % | 26 % | 26 % | 36 % |
| L-Menthol (pur) | 0 % | 59 % | 82 % | 100 % | 100 % | 100 % | 100 % |
| 7 % L-Menthol mit Soluplus Pulver | 0 % | 11 % | 9 % | 26 % | 44 % | 44 % | 58 % |
| 7 % L-Menthol mit Soluplus Granulat | 0% | 0% | 0% | 0% | 11 % | 5% | 11 % |

### Test 2

Verzögerte Freisetzung der Geruchs- und Geschmacksstoffe durch unterschiedliche Auflösungsgeschwindigkeit der Formulierungen:
Einwiegen einer entsprechenden Menge Formulierung (Aromakonzentration entspricht 100% der Sättigungslöslichkeit der Aromastoffe in Wasser) in ein 25 mL Becherglas. 25 mL demineralisiertes Wasser hinzufügen und unter Rühren die Auflösungsgeschwindigkeit (vollständige Auflösung der Formulierung) bei Raumtemperatur bestimmen.

Sättigungslöslichkeit der Geruchs- und Geschmacksstoffe in Wasser:

| | |
|---|---|
| Geraniol Extra | 0,10 g/L entspricht 2,5 mg/25 mL |
| L-Menthol | 0,397 g/L entspricht 9,925 mg/25 mL |

| Formulierung | Einwaage Formulierung in 25 mL demineralisierten Wasser | Auflösungsgeschwindigkeit in Minuten |
|---|---|---|
| 7 % Geraniol Extra mit Soluplus Pulver | 38 mg | 17 |
| 7 % Geraniol Extra mit Soluplus Granulat | 36 mg | 65 |
| 7 % L-Menthol mit Soluplus Pulver | 145 mg | 33 |
| 7 % L-Menthol mit Soluplus Granulat | 142 mg | 85 |

## Patentansprüche

1. Optisch klare feste wasserlösliche Formulierungen von Geruchs- und Geschmacksstoffen, enthaltend mindestens einen synthetisch hergestellten Geruchs- und Geschmacksstoff, in denen die Geruchs- und Geschmacksstoffe homogen in einer Polymermatrix auf Basis eines Pfropfcopolymers aus (i) 50 bis 60 Gew.-% N-Vinyllactam, bevorzugt N-Vinylcaprolactam, (ii) 25 bis 35 Gew.-% Vinylacetat, und (iii) 10 bis 20 Gew.-% eines Polyethers, bevorzugt Polyethylenglykol, mit der Maßgabe, dass die Summe der Komponenten i), ii), und iii) gleich 100 Gew.-% beträgt, eingebettet vorliegen.

2. Wasserlösliche Formulierungen nach Anspruch 1, enthaltend eine Polymermatrix auf Basis eines Pfropfcopolymers aus 13 Gew.-% Polyethylenglykol MG 6000, 57 Gew.-% N-Vinylcaprolactam und 30 Gew.-% Vinylacetat mit einem mittleren Molgewicht von 44.000 Dalton

3. Wasserlösliche Formulierungen nach Anspruch 1 oder 2, enthaltend als Geruchs-und Geschmacksstoffe eine Substanz ausgewählt aus der Gruppe bestehend aus Geraniol, Linalool, Linalylacetat, Pyranol, Geranylacetat, Anisaldehyd, Citral, Citronellal, Lysmeral, Citronellol, Rosenoxid, Tetrahydrolinalool, Hydroxycitronellal, Betaionon, Menthol, etherische Öle, Zimtaldehyd, Anethol, Vanillin, Eugenol, Zimtöl, Carvon, Piperonal und komplexen Parfüm- oder Geschmackskompositionen.

4. Wasserlösliche Formulierungen nach einem der Ansprüche 1 bis 3, enthaltend als Geruchs- und Geschmacksstoffe eine Substanz ausgewählt aus der Gruppe bestehend aus Geraniol, Linalool, Linalylacetat, Pyranol, Geranylacetat, Anisaldehyd, Citral, Citronellal, Lysmeral, Citronellol, Rosenoxid, Tetrahydrolinalool, Hydroxycitronellal, Betaionon, Menthol, Zimtaldehyd, Anethol, Vanillin, Eugenol, Carvon und Piperonal.

5. Wasserlösliche Formulierungen nach einem der Ansprüche 1 bis 4, erhältlich durch Schmelzextrusion.

6. Verwendung der wasserlöslichen Formulierungen nach einem der Ansprüche 1 bis 5 als Wasch- und Geschirrspülmittelzusätze, als Kosmetikzusätze, in Pharmazeutika oder Nahrungsergänzungsmitteln, in Lebensmitteln oder zur Abgabe von Geruchsstoffen in Gebäuden und Fahrzeugen.

## Claims

1. An optically transparent, solid water-soluble formulation of odorants and flavorings comprising at least one synthetically prepared odorant and flavoring, in which the odorants and flavorings are embedded homogeneously in a polymer matrix based on a graft copolymer of (i) 50 to 60 wt% N-vinyllactam, preferably N-vinylcaprolactam, (ii) 25 to 35 wt% vinyl acetate, and (iii) 10 to 20 wt% of a polyether, preferably polyethylene glycol, with the proviso that the sum of components i), ii), and iii) is equal to 100 wt%.

2. The water-soluble formulation according to claim 1, comprising a polymer matrix based on a graft copolymer of 13 wt% polyethylene glycol MW 6000, 57 wt% N-vinylcaprolactam and 30 wt% vinyl acetate, having an average molecular weight of 44 000 daltons.

3. The water-soluble formulation according to claim 1 or 2 comprising, as odorants and flavorings, a substance selected from the group consisting of geraniol, linalool, linalyl acetate, pyranol, geranyl acetate, anisaldehyde, citral, citronellal, lysmeral, citronellol, rose oxide, tetrahydrolinalool, hydroxycitronellal, beta-ionone, menthol, essential oils, cinnamaldehyde, anethole, vanillin, eugenol, cinnamon oil, carvone, piperonal and complex perfume or flavoring compositions.

4. The water-soluble formulation according to any of claims 1 to 3 comprising, as odorants and flavorings, a substance selected from the group consisting of geraniol, linalool, linalyl acetate, pyranol, geranyl acetate, anisaldehyde, citral, citronellal, lysmeral, citronellol, rose oxide, tetrahydrolinalool, hydroxycitronellal, beta-ionone, menthol, cinnamaldehyde, anethole, vanillin, eugenol, carvone and piperonal.

5. The water-soluble formulation according to any of claims 1 to 4, obtainable by melt extrusion.

6. The use of the water-soluble formulations according to any of claims 1 to 5 as additives for detergent and dishwashing compositions, as cosmetic additives, in pharmaceuticals or food supplements, in foods or for releasing odorants in buildings and vehicles.

## Revendications

1. Formulations solubles dans l'eau solides optiquement transparentes de matières olfactives et de matières aromatiques, contenant au moins une matière olfactive et une matière aromatique préparées synthétiquement, dans lesquelles les matières olfactives et les matières aromatiques sont présentes incorporées de manière homogène dans une matrice de polymère à base d'un copolymère greffé composé de (i) 50 à 60 % en poids d'un N-vinyllactame, préférablement de N-vinylcaprolactame, (ii) 25 à 35 % en poids d'acétate de vinyle, et (iii) 10 à 20 % en poids d'un polyéther, préférablement un polyéthylène glycol, à la condition que la somme des composants i), ii), et iii) soit égale à 100 % en poids.

2. Formulations solubles dans l'eau selon la revendication 1, contenant une matrice de polymère à base d'un copolymère greffé composé de 13 % en poids de polyéthylène glycol MG 6000, 57 % en poids de N-vinylcaprolactame et 30 % en poids d'acétate de vinyle comportant un poids moléculaire moyen de 44 000 Daltons.

3. Formulations solubles dans l'eau selon la revendication 1 ou 2, contenant en tant que matières olfactives et matières aromatiques une substance choisie dans le groupe constitué par le géraniol, le linalool, l'acétate de linalyle, le pyranol, l'acétate de géranyle, l'anisaldéhyde, le citral, le citronellal, le lysméral, le citronellol, l'oxyde de rose, le tétrahydrolinalool, l'hydroxycitronellal, la bêta-ionone, le menthol, des huiles essentielles, le cinnamaldéhyde, l'anéthol, la vanilline, l'eugénol, l'essence de cannelle, la carvone, le pipéronal et des compositions complexes de parfum ou d'arôme.

4. Formulations solubles dans l'eau selon l'une quelconque des revendications 1 à 3, contenant en tant que matières olfactives et matières aromatiques une substance choisie dans le groupe constitué par le géraniol, le linalool, l'acétate de linalyle, le pyranol, l'acétate de géranyle, l'anisaldéhyde, le citral, le citronellal, le lysméral, le citronellol, l'oxyde de rose, le tétrahydrolinalool, l'hydroxycitronellal, la bêta-ionone, le menthol, le cinnamaldéhyde, l'anéthol, la vanilline, l'eugénol, la carvone et le pipéronal.

5. Formulations solubles dans l'eau selon l'une quelconque des revendications 1 à 4, qui peuvent être obtenues par extrusion à l'état fondu.

6. Utilisation des formulations solubles dans l'eau selon l'une quelconque des revendications 1 à 5 en tant qu'additifs de lessive et de produits pour laver la vaisselle, en tant qu'additifs de produit cosmétique, dans des produits pharmaceutiques et des suppléments alimentaires, dans des produits alimentaires ou pour l'émission de matières olfactives dans des bâtiments et des véhicules.
